# FASCICULE DE BREVET EUROPEEN

(11) **EP 3 273 947 B1**
(45) Date de publication et mention de la délivrance du brevet: **12.06.2019**
(21) Numéro de dépôt: 16719447.1
(22) Date de dépôt: 25.03.2016
(51) Int. Cl.: A61K 31/05, A61K 31/07, A61K 31/202, A61K 31/355, A61K 31/375, A61K 33/30, A61K 33/34, A61K 9/48, A61P 27/02, A61K 31/047

(54) **COMPOSITION POUR LA SANTE OCULAIRE**
ZUBEREITUNG FÜR DIE GESUNDHEIT DER AUGEN
COMPOSITION FOR OCULAR HEALTH

(30) Priorité: 25.03.2015 FR 1552483
(43) Date de publication de la demande: 31.01.2018
(73) Titulaire: Laboratoires Théa, 63100 Clermont-Ferrand (FR)
(72) Inventeur: OLMIERE, Céline, 63910 Vertaizon (FR); MERCIER, Fabrice, 63000 Clermont-Ferrand (FR)
(74) Mandataire: Cabinet Laurent & Charras
(86) Numéro de dépôt international: PCT/FR2016/050697
(87) Numéro de publication internationale: WO 2016/151269

(56) Documents cités:
- GB-A- 2 483 121
- US-A1- 2005 163 873
- CHANDRASEKHARAM N NAGINENI ET AL: "Resveratrol Suppresses Expression of VEGF by Human Retinal Pigment Epithelial Cells: Potential Nutraceutical for Age-related Macular Degeneration", AGING AND DISEASE, vol. 5, no. 2, avril 2014 (2014-04), pages 88-100, XP055232237, DOI: 10.14366/AD.2014.050088 cité dans la demande
- JAMA INTERNAL MEDICINE: "Effect of Long-Chain omega-3 Fatty Acids and Lutein plus Zeaxanthin Supplements on Cardiovascular Outcomes Results of the Age-Related Eye Disease Study 2 (AREDS2) Randomized Clinical Trial", BONDS DENISE E ET AL , mai 2014 (2014-05), page 17PP, XP002751613, Extrait de l'Internet: URL:http://archinte.jamanetwork.com/articl e.aspx?articleid=1835351 [extrait le 2015-11-30] cité dans la demande

## Description

### DOMAINE DE L'INVENTION

La présente invention propose une composition ophtalmique nutraceutique, notamment pour l'amélioration de la santé oculaire et la prévention des maladies oculaires. Une telle composition comprend des vitamines, des oligoéléments, des caroténoides, des acides gras oméga 3 et du resvératrol en quantité supérieure à 20 mg.

De manière surprenante, la présente demande démontre une meilleure efficacité et un effet synergique du resvératrol dans une telle association, tant dans le traitement des cellules rétiniennes pathologiques que dans le maintien et l'amélioration de la santé des cellules saines.

### ETAT ANTERIEUR DE LA TECHNIQUE

La dégénérescence maculaire liée à l'âge (DMLA) est la première cause de handicap visuel chez les personnes de plus de 50 ans. Toutes formes confondues, cette maladie concerne environ 8 % de la population française, mais sa fréquence augmente largement avec l'âge : elle touche 1 % des personnes de 50 à 55 ans, environ 10 % des 65-75 ans et de 25 à 30 % des plus de 75 ans. Si l'on tient compte uniquement des formes tardives de la maladie, associées à une perte de la vision centrale, ces chiffres sont à diviser environ par deux. Mais dans les années à venir, compte tenu de l'allongement de l'espérance de vie, l'incidence de la DMLA ne va cesser de croitre.

Cette pathologie touche sélectivement la région maculaire, c'est à dire la zone centrale de la rétine, entraînant une perte progressive de la vision centrale à savoir le champ de vision utile pour lire, reconnaître des visages ou conduire. Elle est la première cause de malvoyance chez les sujets âgés.

La maladie débute par une phase précoce, sans dégénérescence, appelée maculopathie liée à l'âge (MLA ou "sèche précoce"). Cette phase se caractérise par l'accumulation de petits dépôts blanchâtres (ou "drusen mous") à l'intérieur et autour de la macula. Ces dépôts sont visibles lors d'un simple examen de fond d'oeil. Cette phase est le plus souvent asymptomatique, mais le patient peut éventuellement percevoir des déformations des lignes droites ("métamorphopsies") et des taches floues.

En effet, à l'examen du fond d'oeil, l'ophtalmologiste observe des taches blanches (drusen) ou des irrégularités de la couche profonde de la rétine, signes caractéristiques de DMLA chez une personne de plus de 50 ans. Lorsque l'examen du fond d'oeil est réalisé à un stade plus avancé, il met en évidence des lésions plus évoluées, telles que des altérations de l'épithélium pigmentaire, des hémorragies ou des dépôts de liquides, aussi appelés exsudats.

Aux stades plus avancés, des symptômes apparaissent, variant en fonction de la gravité de la maladie:
- Diminution de l'acuité visuelle, avec nécessité d'avoir un meilleur éclairage pour la lecture ou pour tout travail de précision ;
- Vision centrale de plus en plus floue, altération de la perception des couleurs, distorsion des lignes droites, qui apparaissent déformées et gondolées ;
- Apparition d'une tache sombre au centre du champ visuel appelée scotome, difficultés à reconnaître les visages, hallucinations visuelles ou baisse brutale de l'acuité visuelle ;
- L'atteinte sévère du deuxième oeil est très variable d'un sujet à l'autre : elle peut survenir rapidement, dans un an, dans 10 ans, ou jamais. Si une DMLA exsudative est présente à un oeil, il y a un risque de la développer au deuxième.

Une MLA peut rester stable tout au long de la vie. Néanmoins, dans environ la moitié des cas et sous l'influence de plusieurs facteurs, la MLA évolue en formes dégénératives tardives :
- Forme atrophique qui est une forme sèche, aussi appelée forme atrophique, dans laquelle la macula s'atrophie en vieillissant et est progressivement remplacée par du tissu cicatriciel ; ou
- Forme humide qui est une forme exsudative dans laquelle de petits vaisseaux gorgés de sang se développent sous la macula. Saignant facilement, ces néovaisseaux sont responsables d'exsudats et d'hémorragies du fond d'oeil.

Ces deux formes tardives ont une incidence à peu près équivalente. Elles conduisent à une dégradation irréversible de la macula et à une perte de la vision centrale affectant un seul oeil ou les deux. Des formes mixtes peuvent être observées.

La forme humide de la DMLA, dite néovasculaire ou exsudative, tout comme la rétinopathie diabétique, l'oedème maculaire, l'occlusion veineuse rétinienne ou encore la forte myopie, se traduit par une prolifération de nouveaux vaisseaux anormaux sous la rétine. Ces vaisseaux fragiles laissent diffuser du sérum, responsable d'un soulèvement de la rétine, et/ou du sang entrainant l'apparition d'hémorragies rétiniennes. Cette forme évolue rapidement si elle n'est pas prise en charge, avec une perte de la vision en quelques semaines ou même quelques jours. Ce processus peut être ralenti par des médicaments de type anti-VEGF.

Ainsi, depuis 2006, la forme humide de la DMLA est traitée à l'aide d'inhibiteurs du VEGF. Progressivement, les autres pathologies néovasculaires (oedème maculaire, occlusion veineuse rétinienne ou forte myopie) sont également traitées à l'aide de ces traitements. Le VEGF est un facteur de croissance qui permet la formation des néo-vaisseaux. Son blocage par des injections répétées d'anti-VEGF, directement dans l'oeil par voie intra vitréenne à raison de sept injections par an en moyenne, permet de stopper la progression de la maladie. Il existe actuellement trois inhibiteurs de VEGF :
- le pegaptanib depuis 2006, considéré comme peu efficace ;
- le ranibizumab depuis 2007, avec environ 30% de non-répondeurs ou d'échappement au traitement ; et
- l'aflibercept depuis 2012.

Ces anti-VEGF ont largement supplanté les anciennes techniques destinées à détruire les néo-vaisseaux, notamment la photocoagulation (destruction thermique des vaisseaux anormaux) et la photodynamique. Cette dernière technique, datant des années 2000, consiste à injecter par voie intraveineuse un produit photosensible (vertéporfine) qui devient toxique sous l'effet de lumière rouge appliquée localement à l'aide d'un laser.

En amont de ces traitements pharmaceutiques lourds, mis en oeuvre à des stades avancés de la pathologie, des études ont été menées pour tenter de trouver des prises en charges plus précoces et plus « légères », ayant pour but de prévenir l'avancée de la maladie.

Ainsi, des études appelées AREDS 1 & 2 (acronyme anglais de "*Age-Related Eye Disease Study*") ont été menées dans le but de démontrer l'effet bénéfique de compléments alimentaires sur les maladies oculaires.

Ces études ont révélé que des suppléments alimentaires à base d'antioxydants étaient potentiellement capables de prévenir le développement de maladies oculaires, notamment celles liées à l'âge, en particulier la DMLA.

En pratique, l'étude AREDS 1 préconise la prise quotidienne d'antioxydants de type oligoéléments et vitamines, comme suit :

| | |
|---|---|
| Zinc | 80 mg |
| Vitamine C | 500 mg |
| Vitamine E | 267 mg |
| Cuivre | 2 mg |
| Béta-carotène (= Vitamine A) | 15 mg |

L'étude AREDS 2 a par ailleurs préconisé la suppression de la vitamine A et rapporté le bénéfice de la prise quotidienne supplémentaire de caroténoïdes (pigments maculaires) et d'acides gras oméga 3, comme suit :

| | |
|---|---|
| Lutéine | 10 mg |
| Zéaxanthine | 2 mg |
| acide docosahexaénoïque (DHA) | 350 mg |
| acide eicosapentaénoïque (EPA) | 650 mg |

Sur la base des résultats des études AREDS, il est donc préconisé la prise quotidienne de tels compléments alimentaires chez les personnes présentant un risque élevé de développer une DMLA, en particulier chez les malades présentant une MLA à l'un ou aux deux yeux, ainsi que chez les patients atteints d'une DMLA avancé ne touchant encore qu'un oeil.

L'article « *Effect of long-chain ω-3 fatty acids and lutein* + *zeaxanthin supplements on cardiovascular outcomes* » du groupe de recherche AREDS2 (JAMA Intern Med. 2014 ; 174(5) :763-771) décrit l'absence d'effets de ce type de compléments alimentaires sur les pathologies cardiovasculaires, lorsque ceux-ci sont administrés à des sujets atteints de DMLA. En tout état de cause, cette étude ne décrit nullement les effets des suppléments alimentaires administrés sur la DMLA, ni sur la santé oculaire de manière générale.

Il existe toutefois un besoin évident de développer des solutions permettant une meilleure prise en charge notamment de la DMLA, tant au niveau de sa prévention que de son traitement.

### EXPOSE DE L'INVENTION

Selon un premier aspect, la présente invention vise une composition ophtalmique nutraceutique comprenant :
- des vitamines ;
- des oligoéléments ;
- des caroténoïdes ;
- des acides gras oméga 3 ; et
- du resvératrol en quantité supérieure à 20 mg.

Dans le cadre de l'invention, on appelle « composition nutraceutique », une composition dont le but est de compléter le régime alimentaire normal et constituant une source concentrée de nutriments ou d'autres substances ayant un effet nutritionnel ou physiologique, seuls ou combinés. Il s'agit avantageusement d'un complément ou supplément alimentaire.

Dans le cadre de l'invention, on appelle « composition ophtalmique », une composition présentant des propriétés bénéfiques pour la santé oculaire. Ceci n'exclut toutefois pas la possibilité qu'une telle composition ait un effet bénéfique au niveau d'autres organes de l'homme ou de l'animal.

Dans la présente demande, il est montré que la combinaison des différents composés listés a une action bénéfique dans le cadre de la modulation, à la fois au niveau de l'expression (modification quantitative) et de la phosphorylation/activité (modification qualitative), des récepteurs aux facteurs de croissance de l'endothélium vasculaire (VEGF pour « *Vascular endothelial growth factor* ») notés VEGFR dans la suite de l'exposé.

Selon une première variante, la composition selon l'invention est constituée des ingrédients listés ci-dessus.

De manière avantageuse, les ingrédients vitamines, oligoéléments, acides gras oméga 3 et caroténoïdes, connus pour leurs propriétés antioxydantes, sont choisis comme préconisés par les études AREDS.

Selon un mode de réalisation particulier, la composition selon l'invention comprend au moins une vitamine, avantageusement une combinaison de vitamines. Il s'agit avantageusement de la vitamine C et/ou de la vitamine E.

Il peut également s'agir des vitamines suivantes :
- nicotinamide ou niacinamide ou vitamine B3 ou PP ;
- pyridoxine, par exemple sous forme de pyridoxine HCl, ou vitamine B6 ;
- riboflavine ou vitamine B2 ;
- thiamine, par exemple sous forme de thiamine nitrate, ou vitamine B1 ;
- cyanocobalamine ou vitamine B12 ;
- acide folique ou vitamine B9 ;
- Vitamine B5 ;
- Vitamine B8 ;
- Vitamine D.

La vitamine C, disponible sous forme d'acide ascorbique ou d'ascorbate de calcium, est avantageusement présente en quantité (dose journalière) comprise entre 50 et 500 mg, par exemple égale à 240 mg ou 120 mg.

La vitamine E ou alpha-tocophérol, par exemple disponible sous forme d'huile à 67%, est avantageusement présente en quantité (dose journalière) comprise entre 10 et 500 mg, par exemple égale à 30 mg.

Selon un autre mode de réalisation, des quantités quotidiennes suivantes, exprimées en mg, peuvent être envisagées pour les vitamines suivantes :
- vitamine B3 ou PP : 1 à 100, par exemple 18 ;
- vitamine B6 : 1 à 10, par exemple 2 ;
- vitamine B2 : 1 à 10, par exemple 1,6 ;
- vitamine B1 : 1 à 10, par exemple 1,4 ;
- vitamine B12 : 0,0005 à 0,01, par exemple 0,001 ;
- vitamine B9 : 0,1 à 1, par exemple 0,2.

Selon un mode de réalisation particulier, la composition selon l'invention ne comprend pas de vitamine A ou bêta-carotène.

Selon un mode de réalisation particulier, la composition selon l'invention comprend au moins un oligoélément, avantageusement une combinaison d'oligoéléments. Il s'agit avantageusement du zinc (Zn) et/ou du cuivre (Cu). Il peut également s'agir des oligoéléments suivants :
- du manganèse, par exemple du sulfate de manganèse anhydre ;
- du sélénium, par exemple du sélénite de sodium ;
- du magnésium, par exemple de l'oxyde de magnésium.

Le zinc (Zn), disponible sous forme d'oxyde de zinc ou de sulfate de zinc (monohydrate), est avantageusement présent en quantité (dose journalière) comprise entre 5 et 100 mg, par exemple égale à 12,5 mg.

Le cuivre (Cu), disponible sous forme de sulfate de cuivre, mono hydrate ou anhydre, est avantageusement présent en quantité (dose journalière) comprise entre 0,2 et 10 mg, par exemple égale à 1 mg.

Selon un autre mode de réalisation, des quantités quotidiennes suivantes, exprimées en mg, peuvent être envisagées pour les oligoéléments suivants :
- du manganèse : entre 1 et 10 mg, par exemple 1 mg ;
- du sélénium : entre 0,01 et 0,1 mg, par exemple 0,025 mg ;
- du magnésium : entre 1 et 50 mg, par exemple 10 mg.

Selon un mode de réalisation particulier, la composition selon l'invention comprend au moins un caroténoïde, avantageusement une combinaison de caroténoïdes. Il s'agit avantageusement de la lutéine et/ou de la zéaxanthine. Il peut également s'agir des caroténoïdes suivantes :
- de la méso-zéaxanthine ;
- du lycopène ;
- de l'astaxanthine.

La lutéine, disponible sous forme de lutéine à 20% en poids, est avantageusement présente en quantité (dose journalière) comprise entre 2 et 50 mg, par exemple égale à 10 mg.

La zéaxanthine, disponible sous forme de zéaxanthine à 5 ou 14% en poids, est avantageusement présente en quantité (dose journalière) comprise entre 0,5 et 10 mg, par exemple égale à 2 mg.

Selon un mode de réalisation particulier, la composition selon l'invention comprend au moins un acide gras polyinsaturé de type oméga 3, avantageusement une combinaison d'acides gras oméga 3. Il s'agit avantageusement de l'acide eicosapentaénoïque (EPA) et/ou de l'acide docosahexaénoïque (DHA). Il peut également s'agir des acides gras oméga 3 suivants :
- de l'acide alpha-linolénique (ALA) ;
- de l'acide docosapentaénoïque (DPA).

Les acides gras oméga 3 mis en oeuvre dans le cadre de la composition selon l'invention peuvent être issus de l'huile de poisson, par exemple de l'huile de poisson à 70% en masse d'acides gras oméga 3, ou d'huile végétale, par exemple de lin. Ces acides gras sont avantageusement présents en quantité (dose journalière) comprise entre 500 et 1000 mg, par exemple égale à 665 mg ce qui représente un apport en huile de poisson de 950 mg. Plus généralement, l'huile de poisson peut être présente en quantité journalière comprise entre 500 et 1000 mg.

Dans ce contexte, l'EPA est avantageusement présent en quantité (dose journalière) comprise entre 100 et 1000 mg, par exemple égale à 380 mg ou 172 mg.

Dans ce contexte, le DHA est avantageusement présent en quantité (dose journalière) comprise entre 100 et 1000 mg, par exemple égale à 190 mg ou 366 mg.

Dans ce contexte, le DPA est avantageusement présent en quantité (dose journalière) inférieure à 100 mg, voire à 50 mg.

De manière caractéristique, une composition selon l'invention comprend en outre du resvératrol en quantité supérieure à 20 mg.

Certes, le resvératrol a été décrit dans l'article de Nagineni et al. (Aging and Disease, 2014 ; 5(2) : 88-100) comme inhibant *in vitro* la surexpression de VEGF induite par les cytokines dans des cellules humaines de l'épithélium pigmentaire de la rétine (cellules HRPE). Toutefois, aucun effet sur les récepteurs au facteur de croissance VEGF (VEGFR) n'est décrit. Bien que l'article évoque un potentiel intérêt nutraceutique de ce composé en rapport avec la DMLA, il n'enseigne nullement qu'un effet synergique sur l'expression des VEGFR peut être obtenu lorsque le resvératrol est combiné à d'autres composés.

Le resvératrol est un composé polyphénolique, dérivé du stilbène, de formule :

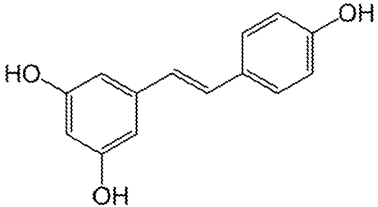

Il existe deux isomères mais la forme *trans* est majoritairement active. Dans la suite de la description, le terme « resvératrol » pourra donc être utilisé en lieu et place de *trans-*resvératrol.

Le resvératrol est un polyphénol retrouvé en quantités abondantes dans certains fruits, notamment dans le raisin et les oléagineux, et dans le vin. Il existe aujourd'hui plusieurs sources commerciales de resvératrol qui peuvent être utilisées pour la préparation d'une composition selon l'invention :
- *Vitis vinifera :*
   ∘ Peau de raisin
   ∘ Pépin de raisin
- *Polygonum Cuspidatum*
- *Produit par fermentation*

Des sources privilégiées sont notamment :
- Resveratrol 5% source *Vitis vinifera,* peau de raisin (PHARMANAGER INGREDIENT) ;
- Resveratrol 98% source *Vitis vinifera,* peau de raisin (CAMBRIDGE COMMODITIES ; CAS Number : 501-36-0).

Le resvératrol est présent en quantité (dose journalière) supérieure à 20 mg, voire supérieure à 30 mg, 50 mg, 60 mg voire même 100 mg ou 200 mg. Selon un mode de réalisation particulier, sa quantité journalière est supérieure ou égale à 30 mg voire supérieure à 30 mg, voire supérieure ou égale à 50 mg, voire encore supérieure ou égale à 60 mg, voire même supérieure ou égale à 100 mg. Avantageusement, elle est inférieure ou égale à 1 g, de préférence inférieure ou égale à 500 mg, de manière particulièrement préférée inférieure ou égale à 200 mg. Selon un mode de réalisation particulier, une composition selon l'invention comprend une dose journalière en resvératrol égale à 50 mg, 60 mg, 70mg, 80 mg, 90 mg, 100 mg, 150 mg ou 200 mg.

A la connaissance du Déposant, il a déjà été préconisé d'utiliser du resvératrol dans des compositions, y compris à visée ophtalmique, en raison de ses propriétés précédemment décrites : antioxydant, anti-apoptotique, anti-tumorigène, anti-inflammatoire, anti-angiogénique et vaso-relaxant.

Ainsi, la demande US 2005/0163873 propose de traiter la composante neurodégénérative du glaucome, à l'aide de compositions ayant un effet protecteur vis-à-vis des dommages des cellules nerveuses. Ce document établit une liste de composés utilisables dans ce contexte dont le resvératrol. Les compositions neuroprotectrices peuvent contenir du resvératrol en combinaison avec divers autres composés. Il n'est pas indiqué explicitement quelle dose journalière de resvératrol pourrait le cas échéant être administrée.

Toutefois, la présente demande met en évidence les nouveaux effets suivants :
- Un effet bénéfique ou du moins non délétère sur des cellules saines ;
- Une activité anti-VEGF, au niveau quantitatif et qualitatif, dans les cellules malades, boostée en présence des autres ingrédients d'une composition selon l'invention, en particulier dans le contexte d'une formulation AREDS.

Selon un autre mode de réalisation, la composition selon l'invention peut contenir d'autres actifs tels que :
- un ou d'autres polyphénol(s), tels que l'épigallocatechine-3-gallate (EGCG) ;
- du glutathion, par exemple à hauteur de 1 à 10 mg par jour, voire 2 mg par jour ;
- un ou des anthocyanoside(s) ;
- de l'hydroxythyrosol.

La teneur en chacun de ces ingrédients est aisément déterminée et ajustée par l'homme du métier.

Ainsi et selon une variante, la composition comprend ou est constituée des ingrédients suivants :
- vitamine C ;
- vitamine E ;
- zinc ;
- cuivre ;
- lutéine ;
- zéaxanthine ;
- acides gras oméga 3 issus de l'huile de poisson, notamment EPA et DHA ;
- resvératrol en quantité supérieure à 20 mg.

Selon une autre variante, la composition comprend ou est constituée des ingrédients suivants, sur la base d'une dose quotidienne:
- de 50 mg à 500 mg de vitamine C, avantageusement 240 mg, de manière particulièrement avantageuse 120 mg ;
- de 10 mg à 500 mg de vitamine E, avantageusement 30 mg ;
- de 5 mg à 100 mg de zinc, avantageusement 12,5 mg ;
- de 0,2 mg à 10 mg de cuivre, avantageusement 1 mg ;
- de 2 mg à 50 mg de lutéine, avantageusement 10 mg ;
- de 0,5 mg à 10 mg de zéaxanthine, avantageusement 2 mg ;
- de 100 mg à 1000 mg de EPA, avantageusement 380 mg, de manière particulièrement avantageuse 172 mg ;
- de 100 mg à 1000 mg de DHA, avantageusement 190 mg, de manière particulièrement avantageuse 366 mg ;
- du resvératrol, en quantité supérieure à 20 mg, de préférence supérieure à 30 mg, de manière particulièrement préférée supérieure à 50 mg, de manière très avantageuse en quantité supérieure ou égale à 60 mg.

Selon un mode de réalisation préféré, la composition de l'invention comprend ou est constituée des ingrédients suivants, sur la base d'une dose quotidienne:
- 120 mg de vitamine C;
- 30 mg de vitamine E;
- 12,5 mg de zinc ;
- 1 mg de cuivre;
- 10 mg de lutéine;
- 2 mg de zéaxanthine;
- 172 mg de EPA;
- 366 mg de DHA;
- du resvératrol, avantageusement en quantité à 50 mg, de manière très avantageuse en quantité supérieure ou égale à 60 mg.

Selon un mode de réalisation avantageux, cette composition est formulée dans 2 capsules, chacune contenant les ingrédients listés ci-dessus en quantité deux fois moindre.

Selon une première alternative, tous ces ingrédients sont mélangés et administrés dans une composition unique. Alternativement, ces ingrédients peuvent être combinés de différentes manières pour être formulés et/ou administrés de manière simultanée ou décalée dans le temps.

Selon un mode de réalisation particulier, la composition selon l'invention est dépourvue d'au moins un des ingrédients de la liste suivante :
- le safran ;
- un agent chélateur des métaux, tels que l'EDTA ou l'acide phytique ;
- un nucléotide ;
- le pycnogénol ;
- un extrait de *ginkgo biloba.*

Selon ce mode de réalisation, la composition mise en oeuvre ne combine pas du resvératrol avec du safran, ou du resvératrol avec un agent chélateur des métaux et un ou plusieurs nucléotides, ou du resvératrol et du pycnogénol et/ou un extrait de *ginkgo biloba.*

Le document GB 2483121 décrit des compositions comprenant systématiquement une combinaison de resvératrol et de safran, et enseigne que ces deux composés possèdent un effet synergique bénéfique sur la progression des pathologies associées à l'âge, notamment la DMLA. Dans ce document, le safran est indiqué comme connu pour avoir, à lui seul, un effet sur la DMLA. Le resvératrol est quant à lui présenté comme ayant des vertus anti-âge de manière générale, qui découleraient de ses propriétés antioxydantes et anti-inflammatoires.

Selon un aspect particulier, l'invention concerne une composition ophtalmique nutraceutique comprenant :
- des vitamines ;
- des oligoéléments ;
- des caroténoïdes ;
- des acides gras oméga 3 ; et
- du resvératrol en quantité supérieure à 20 mg ;
ladite composition ne comprenant pas de safran,
et l'utilisation d'une telle composition en tant que complément alimentaire en ophtalmologie.

Une composition selon l'invention est avantageusement destinée à une administration par voie orale.

Elle peut se présenter sous forme liquide, de solution, de suspension, de pâte ou de gel.

Avantageusement, elle se présente sous forme solide, telle que poudre, tablettes y compris tablettes effervescentes, capsules, gélules, comprimés ou pastilles, préparés de manière conventionnelle à l'aide d'additifs acceptables tels que des agents liants, des agents de comblement, des lubrifiants, des agents de désintégration ou des agents mouillants. Les tablettes peuvent optionnellement être « coatées » de manière connue de l'homme du métier à l'aide de sucres, de films ou de revêtements entériques.

De manière alternative, d'autres systèmes de délivrance peuvent être mis en oeuvre, tels que des préparations dans de la gélatine molle ou des capsules à base de gélatine ou des formulations préparées à l'aide des nanotechnologies, telles que des nano-dispersions, nano-émulsions ou nano-encapsulations.

La composition selon l'invention peut en outre contenir un ou plusieurs additifs tels que colorants, pigments, arômes, ...

Selon un mode de réalisation particulier, la composition se présente sous forme de capsules, définissant un volume donné dans lequel est contenue la composition. La capsule se décompose dans l'appareil digestif afin de libérer les principes actifs et permettre son assimilation par l'organisme.

De manière connue de l'homme du métier, la répartition dans ce type de conditionnement relève d'un compromis entre :
- un nombre limité de prises, pour assurer une bonne observance du traitement ;
- un volume adapté des prises, permettant la formulation de l'ensemble des ingrédients et une ingestion aisée.

Dans le cas présent, une composition selon l'invention se présente préférentiellement sous la forme d'une ou plusieurs capsules, avantageusement 2 capsules. Ainsi, ces capsules peuvent être prises avec un peu d'eau en début ou au milieu du repas principal. De manière préférentielle, une composition selon l'invention est administrée de manière quotidienne ou journalière, par exemple une fois par jour.

Dans ce contexte, des additifs classiques sont :
- du glycérol, par exemple du glycérol monostéarate ; et/ou
- de l'huile de soja ; et/ou
- de la cire d'abeille ; et/ou
- de la lécithine de soja.
De manière adaptée, ces additifs sont ajoutés à une composition selon l'invention en raison de leur capacité à améliorer par exemple les propriétés d'écoulement ou l'homogénéisation de celle-ci.

En outre et de manière connue de l'homme du métier, la tunique des capsules peut être élaborée à partir des ingrédients suivants :
- gélatine ; et/ou
- oxyde de fer rouge ; et/ou
- oxyde de fer noir ; et/ou
- sorbitol ; et/ou
- glycérol.

Dans le cadre de la présente invention, il a été constaté que l'activité anti-VEGF du resvératrol pouvait être améliorée, aussi bien au niveau de l'inhibition de l'expression des récepteurs que de leur phosphorylation, par la présence d'autres ingrédients, en l'occurrence vitamines, oligoéléments, caroténoïdes et acides gras oméga 3, non connus pour cette propriété. De manière remarquable, cet effet est dose-dépendant.

Ainsi, la composition selon l'invention présente un intérêt évident pour son utilisation dans le traitement des pathologies oculaires associées à une surexpression ou surproduction de la voie VEGF / VEGFR (récepteurs au VEGF), telles que la DMLA, en particulier exsudative, l'occlusion veineuse rétinienne, l'oedème maculaire, l'oedème maculaire diabétique, la myopie, la rétinopathie diabétique, le glaucome néovasculaire ou la néovascularisation cornéenne.

Selon un autre aspect, la présente invention concerne une composition telle que définie ci-dessus pour son utilisation dans:
- le maintien ou l'amélioration de la santé oculaire ; et/ou
- la prévention des maladies oculaires ; et/ou
- la modulation de l'expression et/ou de l'activité des récepteurs au VEGF (VEGFR) des cellules oculaires, telles que rétiniennes ou du segment antérieur (cornéennes).

Si l'effet du resvératrol en tant qu'agent anti-VEGF sur des cellules rétiniennes était connu, son utilisation sur des cellules saines laissait craindre un effet comparable, possiblement préjudiciable à la santé oculaire (Pournaras et al., Progress in Retinal and Eye Research 27 (2008) 284-330). Or, dans le cadre de la présente demande, il a été montré qu'une composition selon l'invention comprenant du resvératrol avait non seulement un effet anti-VEGF sur les cellules pathologiques mais avait, au contraire de ce qui était attendu par l'homme du métier, un effet non délétère, voire bénéfique sur les cellules non atteintes.

Ainsi et notamment, une composition selon l'invention peut bénéfiquement être administrée à un sujet présentant au moins certains des symptômes des pathologies listées ci-dessus uniquement au niveau d'un oeil. De manière inattendue, la composition selon l'invention permet non seulement de traiter l'oeil malade mais ne présente aucun risque pour l'oeil non affecté, voire permet d'assurer ou de prolonger sa bonne santé.

Ainsi, l'invention vise de préférence la composition selon l'invention pour les utilisations susmentionnée, caractérisée en ce qu'elle est utilisée chez un sujet atteint des symptômes de la pathologie uniquement au niveau d'un oeil, un sujet sain, ou un sujet jeune à risque.

Selon un autre aspect, la présente invention concerne un procédé pour le maintien et/ou l'amélioration de la santé oculaire chez un sujet, consistant à administrer, par voie orale, une composition selon l'invention.

Dans le cadre de l'invention, les termes « sujet » et « patient » peuvent être utilisés indifféremment.

Avantageusement, le sujet est un sujet sain, dont l'examen ophtalmologique ne révèle aucun des signes caractéristiques des pathologies oculaires, en particulier MLA ou DMLA. Encore plus avantageusement, il s'agit d'un sujet dit «jeune », c'est-à-dire âgé de moins de 50 ans, voire de moins de 45 ans. Selon un mode de réalisation particulier, il s'agit d'un sujet jeune dit « à risque », c'est-à-dire présentant des caractéristiques ou des antécédents associés à un risque accru de développer une pathologie oculaire, en particulier une MLA ou une DMLA. Les facteurs à risque aujourd'hui identifiés sont en particulier :
- fumeur ;
- risque génétique héréditaire ;
- hypertension artérielle ;
- obésité ;
- opération de la cataracte ;
- myopie ;
- diabète.

Selon un autre aspect, la présente invention concerne un procédé pour prévenir les maladies oculaires chez un sujet, consistant à administrer, par voie orale, une composition selon l'invention.

Avantageusement, les maladies oculaires visées sont les maladies liées à l'âge, en particulier les atteintes rétiniennes, et encore plus précisément les pathologies rétiniennes néovasculaires telles que la DMLA exsudative.

Préférentiellement, le sujet est un sujet sain comme défini ci-dessus, ou un sujet atteint de certains de ces signes uniquement au niveau d'un oeil.

Selon un autre aspect, la présente invention concerne un procédé pour moduler l'expression et/ou l'activité des récepteurs VEGFR des cellules oculaires chez un sujet, consistant à administrer, par voie orale, une composition selon l'invention. Comme démontré dans la présente demande, la modulation correspond à une inhibition en présence de cellules malades, notamment dans le cadre d'atteintes rétiniennes, alors qu'elle correspond à une préservation voire une stimulation ou activation en présence de cellules saines. Selon un mode de réalisation particulier, le sujet est un sujet atteint d'une pathologie ophtalmique, en particulier néovasculaire, par exemple la DMLA (avantageusement exsudative), l'occlusion veineuse rétinienne, l'oedème maculaire, l'oedème maculaire diabétique, la myopie, la rétinopathie diabétique, le glaucome néovasculaire ou la néovascularisation cornéenne.

Selon un mode de réalisation privilégié, la composition comprenant du resvératrol selon l'invention est une formulation de type AREDS associant d'autres actifs, comme décrit ci-dessus.

Comme déjà dit, selon des études menées par le Déposant, une composition selon l'invention peut favoriser le maintien en bonne santé des cellules rétiniennes saines, tandis qu'elle contribue à éliminer les cellules rétiniennes pathologiques. Ainsi, une telle composition peut être préconisée dans le cas de patients atteints d'une pathologie ophtalmique, en particulier néovasculaire, telle que listée ci-dessus, ou atteints d'une telle pathologie à un seul oeil.

Outre cet effet bénéfique dans le cas d'une pathologie détectable ou déclarée, une composition selon l'invention peut être utilisée pour maintenir ou stimuler l'expression et/ou l'activité (la phosphorylation) des récepteurs au VEGF (VEGFR) des cellules oculaires, notamment rétiniennes, saines. Cela a pour conséquence une bonne néovascularisation, et donc une bonne irrigation sanguine avec un apport accru d'éléments nutritifs essentiels pour ces cellules, de manière à les maintenir en bonne santé et à prévenir les maladies, notamment celles liées à l'âge.

Une population cible est donc constituée par des sujets dont l'examen ophtalmologique ne révèle aucun des signes caractéristiques d'une pathologie oculaire néovasculaire. Comme déjà dit, il peut s'agir d'individus dits «jeunes », c'est-à-dire âgés de moins de 50 ans, voire de moins de 45 ans. En particulier, il peut s'agir d'individus dits « à risque », présentant au moins l'une des caractéristiques suivantes :
- fumeur ;
- risque génétique héréditaire ;
- hypertension artérielle ;
- obésité ;
- opération de la cataracte ;
- myopie ;
- diabète.

### EXEMPLES DE REALISATION DE L'INVENTION

L'invention et les avantages qui en découlent ressortiront mieux des figures et exemples suivants donnés, afin d'illustrer l'invention et non de manière limitative.

### DESCRIPTION DES FIGURES

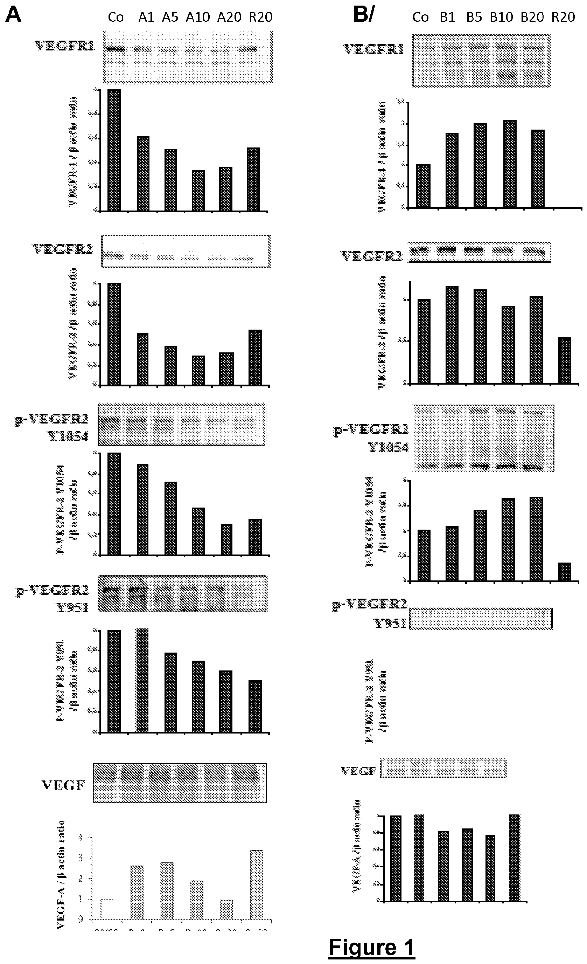

La figure 1 compare, dans des cellules rétiniennes indifférenciées (pathologiques) incubées (A) en présence d'une composition selon l'invention (formule A) ou (B) en présence d'une composition similaire à celle de l'invention mais dépourvue de resvératrol (formule B), l'expression des récepteurs VEGFR (VEGFR1 et VEGFR2) et leur état de phosphorylation (p-VEGF2 Y1054 et Y951), ainsi que du VEGF, par western blot (image) et normalisation à l'aide de la β-actine (diagramme).

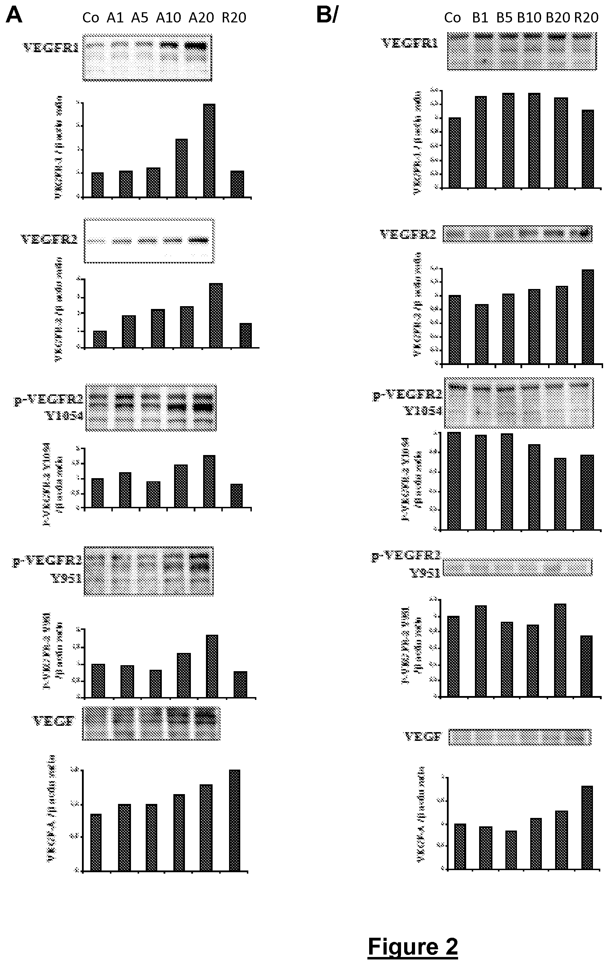

La figure 2 compare, dans des cellules rétiniennes différenciées (saines) incubées (A) en présence d'une composition selon l'invention (formule A) ou (B) en présence d'une composition similaire à celle de l'invention mais dépourvue de resvératrol (formule B), l'expression des récepteurs VEGFR (VEGFR1 et VEGFR2) et leur état de phosphorylation (p-VEGF2 Y1054 et Y951), ainsi que du VEGF, par western blot (image) et normalisation à l'aide de la β-actine (diagramme).

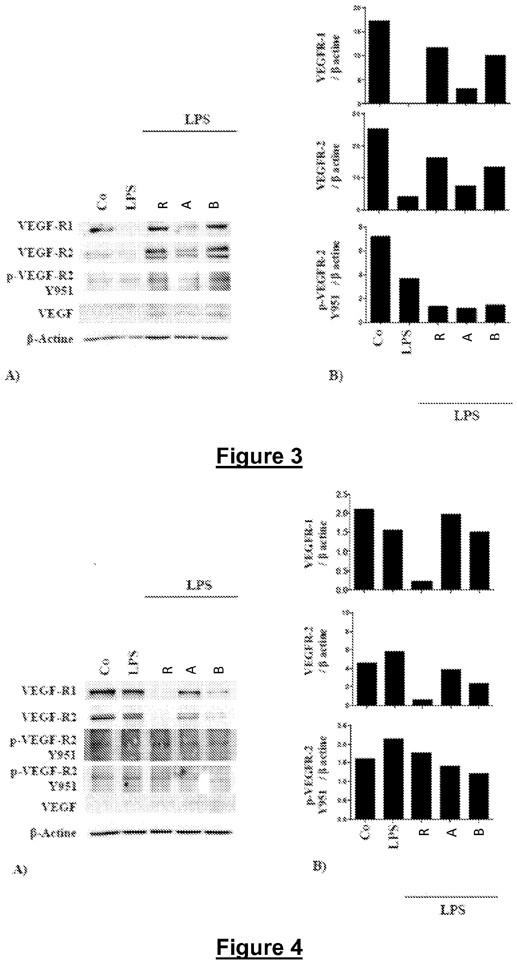

La figure 3 compare par western blot (A) et sous forme de diagramme après normalisation à l'aide de la β-actine (B) l'expression des récepteurs VEGFR (VEGFR1 et VEGFR2) et leur état de phosphorylation (p-VEGF2 Y951) dans l'oeil de souris ayant ou non (Co) reçu une injection de LPS (LPS), lorsque celles-ci sont traitées à l'aide d'une composition selon l'invention (A), de resvératrol seul (R) ou d'une composition dépourvue de resvératrol (B).
La figure 4 compare par western blot (A) et sous forme de diagramme après normalisation à l'aide de la β-actine (B) l'expression des récepteurs VEGFR (VEGFR1 et VEGFR2) et leur état de phosphorylation (p-VEGF2 Y951) dans l'oeil de souris contrôle (Co) ou ayant reçu une injection de LPS mais dans l'oeil controlatéral (LPS), lorsque celles-ci sont traitées à l'aide d'une composition selon l'invention (A), de resvératrol seul (R) ou d'une composition dépourvue de resvératrol (B).

### I/ Composition selon l'invention

### I-1/ Formule de la composition (A)

**Tableau 1 : formule d'une composition selon l'invention (A)**

| ***Ingrédient*** | ***Quantité (mg)* /*jour*** |
|---|---|
| Vitamine C | 240 |
| Vitamine E | 30 |
| Zinc | 12,5 |
| Cuivre | 1 |
| Lutéine | 10 |
| Zéaxanthine | 2 |
| Huile de poisson* | 950 |
| dont DHA | 190 |
| dont EPA | 380 |
| Resvératrol | 30 |

| | |
|---|---|
| *Huile de poisson comprenant 70% en poids d'acide gras oméga 3 dont EPA et DHA. | |

### I-2/ Conditionnement de la composition sous forme de capsules

Les quantités présentées dans le tableau 1 correspondent à la dose journalière. En pratique, la composition selon l'invention est conditionnée sous forme de 2 capsules.

Pour chaque capsule, il est ajouté au mélange-maître :
- glycérol monostérate ;
- cire d'abeilles ;
- lécithine de soja.

La tunique d'une capsule présente la composition suivante :
- Gélatine bovine
- oxyde de fer rouge
- oxyde de fer noir.

Les capsules sont ensuite recouvertes d'une coque en PVC/PVDC et d'un film d'aluminium. De manière alternative, elles peuvent être conditionnées en pilulier.

De manière recommandée, ces deux capsules sont prises avec un peu d'eau en début ou au milieu du repas principal.

### II/ Etude in vitro de la composition selon l'invention (Formule A) :

### II-1/Préparation de solutions à différentes concentrations en resvératrol

La solution mère est préparée dans du DMSO (DiMéthyl SulfOxyde), à raison de 0,115g de la formule A dans 1 ml de DMSO, ce qui correspond à 10 mM en resvératrol.

Des solutions à 1, 5, 10 et 20 µM en équivalent resvératrol, notées A1, A5, A10 et A20 respectivement, sont préparées à partir de cette solution mère. Les dilutions sont réalisées dans un milieu de culture composé de DMEM/F12 + 1% de SVF (Sérum de veau foetal sans phénol rouge).

### II-2/ Effet de la composition selon l'invention sur des cellules pathologiques

### A/ Protocole :

### - Cellules étudiées : culture de cellules ARPE-19 (ATCC® CRL-2302™) («human retinal pigment epithelium ») indifférenciées.

Ces cellules peuvent être assimilées à des cellules rétiniennes atteintes ou pathologiques.

### - Protocole expérimental :

### a) Culture cellulaire :

Les cellules ont été maintenues à 37°C, et en présence de CO2 5% dans du milieu DMEM/F12 (GIBCO; 31331), complété avec 10% FBS (Lonza) et 15 mM d'HEPES (pH 7.4; GIBCO).

### b) Incubation entre les compositions (de formule A ou B) et les cellules :

1, 5, 10 ou 20µM. Le 10 µM de resvératrol correspondant à 0,115 mg/ml de la formule A dans du DMEM/F12 + 1% FBS w/o rouge phénol
L'équivalent 10µM de la formule B correspondant également à 0,115 mg/ml de formule B dans du DMEM/F12 + 1% FBS w/o rouge phénol
Le jour précédent le traitement, le milieu a été éliminé et remplacé par du DMEM/F12 sans rouge phénol (GIBCO; 21041), complété avec 1% FBS (Lonza) et 15 mM d'HEPES (pH 7.4; GIBCO). Après 24h, les cellules ont été traitées pendant 24h avec les différentes formules (Formule A, Formule B et resvératrol) dans du milieu DMEM/F12 sans rouge phénol (GIBCO; 21041), complété avec 1% FBS (Lonza) et 15 mM de HEPES (pH 7.4; GIBCO).

### c) Western blot :

Après 24h de stimulation, les milieux ont été éliminés, les cellules ont été lavées avec du PBS 1X puis grattées dans du PBS 1x. Les cellules ont été centrifugées et le culot a été resuspendu dans du tampon RIPA complété avec un cocktail d'inhibiteurs de protéases (Roche) et des inhibiteurs de phosphatases. Les cellules ont été lysées dans la glace pendant 30 min puis centrifugées. La concentration protéique a été déterminée par un kit de quantification des protéines (Lowry kit, biorad). 45 µg de protéines ont été mélangées avec un volume égal de tampon Laemmli 5x concentré, dénaturées puis chargées sur un gel d'acrylamide. Après électrophorèse, les protéines ont été transférées sur une membrane de nitrocellulose (Amersham). Les blots ont ensuite été bloqués avec du lait à 5% + PBS-T 0.1% pendant 1 heure à température ambiante. Après blocage, les membranes ont été incubées avec des anticorps primaires de Cell Signaling (VEGFR-1, VEGFR-2 (55B11) ou Phospho-VEGF Receptor 2 (Tyr951) (15D2)) ou Abcam (Anti-VEGF antibody [VG-1]). Les membranes ont été lavées 3 fois avec du PBS-T 0.1% puis incubées avec des anticorps conjugués HRP correspondants pendant 1 heure à température ambiante. Les membranes ont à nouveau été lavées 3 fois dans du PBS-T 0.1% et le signal a été détecté à l'aide d'ECL (Tebu bio). Les images ont été enregistrées avec le système d'imagerie ChemiDoc et analysées avec le logiciel Quantity One Software (Biorad).

### d) Quantification du niveau d'expression et d'activité des récepteurs :

La β-actine a également été quantifiée afin de relativiser les niveaux d'expression.

### e) Contrôles :

- Co = échantillon contrôle correspondant au DMSO (servant à la solubilisation des formules) dans le milieu de culture.
- R20 = resvératrol seul à 20µM dilué dans le milieu de culture (= DMEM/F12 + 1% de SVF (Sérum de veau foetal sans phénol rouge).

### B/ Résultats :

### 1) Mise en évidence de l'effet du resvératrol

Pour mettre en évidence l'effet du resvératrol sur l'expression des récepteurs VEGFR, la composition selon l'invention (formule A) est comparée à une composition caractérisée par l'absence de resvératrol, de formule B donnée dans le tableau 2 ci-dessous :

**Tableau 2 : composition de la formule B dépourvue de resvératrol**

| ***Ingrédient*** | ***Quantité (mg)*** |
|---|---|
| Vitamine B1 | 1,4 |
| Vitamine B2 | 1,6 |
| Vitamine B3 | 18 |
| Vitamine B6 | 2 |
| Vitamine B9 | 0,2 |
| Vitamine B12 | 0,001 |
| Vitamine C | 60 |
| Vitamine E | 10 |
| Zinc | 7,5 |
| Manganèse | 1 |
| Sélénium | 0,025 |
| Cuivre | 1 |
| Lutéine | 10 |
| Zéaxanthine | 2 |
| Huile de poisson | 400 |
| dont EPA | 160 |
| | |
| Glutathion | 1 |

Des dilutions de cette composition, notées B1, B5, B10 et B20, ont été réalisées dans des conditions similaires à celles décrites au point II-1 : 0,115g de la formule B dans 1 ml de DMSO puis des dilutions équivalentes dans le milieu de culture.

La figure 1 illustre les résultats obtenus sur des cellules pathologiques, traitées à l'aide de la composition selon l'invention (A) ou de son équivalent sans resvératrol (B).

Il ressort de cette figure que sur ce type de cellules, la présence de resvératrol entraine une diminution de l'expression des récepteurs (VEGFR1 et VEGFR2), ainsi que de leur phosphorylation :
- la composition selon l'invention (A) permet de réprimer l'expression du VEGF et des récepteurs aux VEGF, et ce de manière dose-dépendante ;
- la phosphorylation des récepteurs présents diminue également, et ce de manière dose dépendante, ce qui indique une réduction de l'activation de ces récepteurs.

La composition selon l'invention entraine donc une réduction quantitative, mais aussi qualitative de l'activation des récepteurs aux VEGF dans les cellules pathologiques et par conséquent, de la néovascularisation.

Dans la mesure où la composition dépourvue de resvératrol (formule B) ne permet pas de réduire l'expression des récepteurs aux VEGF, ni leur phosphorylation (Figure 1B), cet effet serait donc lié à la présence du resvératrol.

### 2) Mise en évidence d'une synergie dans la composition selon l'invention

L'effet dose-dépendant de la composition selon l'invention sur des cellules pathologiques a été comparé à celui de la solution R20, contenant uniquement du resvératrol à 20 µM.

La figure 1A révèle que le resvératrol seul permet de diminuer l'expression des récepteurs aux VEGF. Cependant, cet effet est amplifié en présence des autres ingrédients de la composition selon l'invention (comparer A20 et R20).

### II-3/ Effet de la composition selon l'invention sur des cellules saines

### A/ Protocole :

Le protocole mis en oeuvre est le même que celui décrit à l'exemple *II-2,* à l'exception des cellules étudiées:

### - Cellules étudiées : culture de cellules ARPE-19 (human retinal pigment epithelium) différenciées.

### - Protocole d'obtention des cellules différenciées :

Les cellules indifférenciées sont ensemencées dans des flacons T75. Les cellules sont maintenues à 37°C, en présence de CO2 5% dans du milieu DMEM/F12 (GIBCO; 31331), complété avec 10% FBS (Lonza) et 15 mM de HEPES (pH 7.4; GIBCO). Lorsque les cellules arrivent à confluence, le milieu est remplacé par du DMEM/F12 (GIBCO; 31331), complété avec 1% FBS (Lonza) et de l'HEPES (15 mM pH 7.4; GIBCO). Les milieux ont été changés 2 fois par semaine pendant 8 semaines.

Ces cellules peuvent être assimilées à des cellules rétiniennes non atteintes ou saines.

### B/ Résultats :

### 1) Mise en évidence de l'activité du resvératrol

La figure 2 illustre l'effet de la composition selon l'invention (A) ou de son équivalent sans resvératrol (B) sur des cellules saines.

Au contraire de ce qui a été observé sur des cellules pathologiques, la composition selon l'invention (A) augmente l'expression des récepteurs aux VEGF de manière dose-dépendante. De plus, la phosphorylation des récepteurs présents augmente également. Il y a donc une augmentation quantitative mais également qualitative de l'activation, et par conséquent de la néovascularisation des cellules saines.

En revanche, aucun effet n'est observé avec la formulation sans resvératrol (voir Figure 2: comparer Fig. 2A et 2B).
Il ressort de la figure 2 que cette augmentation est dose-dépendante et significative pour les plus hautes concentrations en resvératrol.

### 2) Mise en évidence d'une synergie dans la composition selon l'invention

L'effet dose-dépendant de la composition selon l'invention sur des cellules saines a été comparé à celui d'une solution contenant uniquement du resvératrol à 20 µM (Fig. 2, R20) : celle-ci révèle que le resvératrol seul, à la concentration testée n'a pas d'effet sur les cellules saines, tout comme la formule dépourvue de resvératrol (Fig. 2B).

### III/ Effet de la composition selon l'invention in vivo chez la souris

Afin de valider les résultats précédents obtenus *in vitro* sur un modèle cellulaire, des expériences ont été réalisées chez l'animal, en utilisant comme modèle des souris C57BL/6. Afin de reproduire les symptômes de la DMLA, les souris (hormis le groupe de souris « contrôle ») ont reçu une injection de 0,5 µg d'un extrait de lipopolysaccharides (LPS) dans l'un des 2 yeux (oeil droit).

La DMLA est caractérisée par une inflammation chronique. Les lipopolysaccharides sont bien connus pour induire une inflammation locale, stimulant par conséquent l'expression de cytokines dans les tissus atteints. Attendu que l'administration de LPS est réalisée par injection, et que celle-ci est circonscrite à l'oeil, il est attendu que le LPS n'agit que localement, dans l'oeil injecté (droit), et ne diffusent pas à d'autres tissus, en particulier dans l'oeil gauche.
Préalablement à l'injection de LPS et pour tester l'efficacité des différentes compositions, les souris ont reçu *per os,* une fois par jour et pendant 15 jours, soit du resvératrol seul (R), soit la composition A selon l'invention décrite au tableau 1 (A), soit la composition B comparative dépourvue de resvératrol et décrite au tableau 2 (B).

A l'issue des traitements, les rétines des yeux des souris sont prélevées et les niveaux d'expression des récepteurs au VEGF, VEGF-R1 et VEGF-R2, et de la forme activée (phosphorylée sur la tyrosine 951) du VEGF-R2 ont été analysés.

### A/ Protocole :

Des souris C57BL/6 ont été séparées en 5 groupes :
- 1 groupe contrôle (Co) a reçu uniquement le véhicule ;
- 1 groupe (LPS) a reçu une injection rétro-orbitale de 0,5 µg de LPS *(E. Coli)* dans l'oeil droit au 12ème jour ;
- 1 groupe (R) a reçu, par administration orale, le resvératrol seul à une dose de 35 mg/kg/jour durant 15 jours, et 0,5 µg de LPS ont été administrés dans l'oeil droit par injection rétro-orbitale au 12^{ème} jour, soit 72 h avant la fin de l'expérience;
- 1 groupe (A) a reçu, par administration orale, la composition A du tableau 1 à une dose de 314,5 mg/kg/jour durant 15 jours, et 0,5 µg de LPS ont été administrés dans l'oeil droit par injection rétro-orbitale au 12^{eme} jour, soit 72 h avant la fin de l'expérience;
- 1 groupe (B) a reçu, par administration orale, la composition B du tableau 2 à une dose de 314,5 mg/kg/jour durant 15 jours, et 0,5 µg de LPS ont été administrés dans l'oeil droit par injection rétro-orbitale au 12^{ème} jour, soit 72 h avant la fin de l'expérience.

A la fin de l'expérience, les souris sont anesthésiées et euthanasiées afin de prélever les rétines de l'oeil droit (ayant reçu l'injection de LPS et mimant l'oeil malade) et de l'oeil gauche (mimant l'oeil sain). Puis les rétines sont lysées afin d'étudier par western-blot l'expression des protéines de la voie du VEGF.

Parallèlement, différents organes ont été prélevés pour vérifier la non toxicité des compositions testées.

### B/ Résultats :

Les résultats obtenus pour les rétines de l'oeil droit (mimant l'oeil atteint) sont présentés à la figure 3. Les résultats obtenus pour les rétines de l'oeil gauche (mimant l'oeil sain) sont présentés à la figure 4.

### 1/ Œil traité par injection de LPS (droit) :

Dans l'oeil droit, il est attendu que l'injection de LPS entraîne une réponse inflammatoire, notamment la surexpression de cytokines et donc l'activation de la voie VEGF, comme décrit par Nagineni et al. (Aging and Disease, 2014 ; 5(2) : 88-100). Or, il ressort de la figure 3 que les niveaux d'expression de VEGF-R1, VEGF-R2, la présence de VEGF-R2 sous forme phosphorylée, et même le niveau de VEGF sont très faibles dans les souris notées LPS alors qu'une expression plus importante de ces protéines (en comparaison avec le contrôle non traité Co) était attendue du fait de l'inflammation induite. Ces résultats tendent à montrer qu'un traitement par 0,5 µg de LPS (témoin LPS de la figure 3) est très toxique et très mal supporté par les rétines des souris. La faible quantité de protéines s'explique donc par la toxicité du LPS qui entraine la mort cellulaire et par conséquent les cellules ne peuvent exprimer les récepteurs au VEGF ni secréter le VEGF.

En comparaison, les rétines de l'oeil droit des souris ayant reçu une injection de LPS mais ayant consommé soit du resvératrol (R), soit la composition A, soit la composition B, montrent des niveaux de protéines plus élevés, ce qui semble indiquer que la consommation de ces produits a permis, au moins dans une certaine mesure, de protéger les cellules de la rétine de l'effet toxique des LPS.

En outre, on constate que la prise journalière, pendant 15 jours, de la composition A provoque une diminution significative de l'expression des récepteurs au VEGF tels que VEGF-R1, VEGF-R2 et sa forme activée phospho-VEGF-R2 Y951 comparativement aux souris contrôles, et ce de manière plus significative que ce qui est observé avec la composition B dépourvue de resvératrol ou avec le resvératrol seul (R). Ces résultats sont donc cohérents avec ce qui a été observé *in vitro* à la figure 1, montrant l'intérêt de la composition selon l'invention pour inhiber plus efficacement la voie d'activation des récepteurs au VEGF dans un contexte pathologique, inflammatoire.

### 2/ Œil controlatéral non traité par injection de LPS (gauche) :

Les résultats obtenus avec les rétines de l'oeil gauche, n'ayant pas été en contact avec le LPS et mimant un oeil sain, sont montrés à la figure 4.

Les données obtenues montrent que le resvératrol seul (R), de manière attendue, a pour effet de diminuer l'expression de VEGF-R1 et de VEGF-R2. De manière notable, la composition A selon l'invention permet de maintenir un niveau de VEGF-R1, VEGF-R2 et de la forme activée phospho-VEGF-R2 Y951, comparable aux souris contrôles (Co). L'effet est moindre pour la composition B dépourvue de resvératrol. En d'autres termes et comme observé *in vitro* (figure 2), la combinaison du resvératrol et des vitamines, oligoéléments, caroténoïdes et acides gras oméga 3 de la composition selon l'invention possède un effet synergique en comparaison avec le resvératrol seul et la composition B.

Il ressort des résultats ci-dessus que la composition A est capable de diminuer la voie d'activation du VEGF-R dans les cellules rétiniennes malades mais est également capable de maintenir un niveau normal de fonctionnement de cette voie dans les rétines normales, ce qui est primordial pour maintenir un flux sanguin suffisant vers la rétine et éviter des processus de dégénérescence.

Ainsi et contrairement au resvératrol seul, la composition A peut avantageusement être administrée à des sujets dont un seul des deux yeux est atteints, avec un bénéfice sur l'oeil atteint sans affecter l'oeil sain.

### CONCLUSIONS :

De manière surprenante et pour la première fois dans le domaine de l'ophtalmologie, en rapport avec des cellules rétiniennes, il a été montré qu'une composition selon l'invention, contenant du resvératrol en quantité supérieure à 20 mg, a une action différentielle au niveau des récepteurs VEGFR nécessaires à la néovascularisation, en fonction du type de cellules : répression dans les cellules pathologiques et pas de répression, voire un effet inverse dans les cellules saines.

Ainsi, le resvératrol dans le contexte d'une composition selon l'invention permettrait de rétablir un équilibre dans les cellules « déficientes » mais ne modifierait en rien, et permettrait même de « maintenir en forme » les cellules saines par apport supplémentaire en éléments essentiels apportés par la circulation sanguine.

De la même manière chez les patients à risque, l'administration de la composition A permettrait de prévenir l'apparition des symptômes, en permettant de traiter dès les premières « anomalies » tout en préservant le potentiel santé dans l'attente de cette apparition d'anomalies. En effet, les cellules rétiniennes recevront tout de même l'apport en oxygène, nutriments et vitamines nécessaires par la voie sanguine physiologique entretenue par un taux physiologique de VEGF, qui ne sera pas abaissé comme ce pourrait être le cas avec le resvératrol seul.

## Revendications

1. Composition ophtalmique nutraceutique comprenant :
- des vitamines ;
- des oligoéléments ;
- des caroténoïdes ;
- des acides gras oméga 3 ; et
- du resvératrol en quantité supérieure à 20 mg ;
ladite composition ne comprenant pas de safran.

2. Composition selon la revendication 1, ***caractérisée* en ce qu'**elle comprend une quantité supérieure ou égale à 30 mg de resvératrol, avantageusement supérieure à 30 mg, encore plus avantageusement supérieure ou égale à 50 mg.

3. Composition selon l'une des revendications précédentes, ***caractérisée* en ce que**:
- les vitamines, seules ou en combinaison, sont choisies dans le groupe comprenant la vitamine C et la vitamine E ;
- les oligoéléments, seuls ou en combinaison, sont choisis dans le groupe comprenant le zinc et le cuivre ;
- les caroténoïdes, seuls ou en combinaison, sont choisis dans le groupe comprenant la zéaxanthine et la lutéine ;
- les acides gras oméga 3, seuls ou en combinaison, proviennent de l'huile de poisson et sont avantageusement choisis dans le groupe comprenant l'acide eicosapentaénoïque (EPA) et l'acide docosahexaénoïque (DHA).

4. Composition selon l'une des revendications précédentes, ***caractérisée* en ce qu'**elle comprend :
- vitamine C ;
- vitamine E ;
- zinc ;
- cuivre ;
- lutéine ;
- zéaxanthine ;
- acides gras oméga 3 issus de l'huile de poisson, notamment EPA et DHA.

5. Composition selon l'une des revendications précédentes, ***caractérisée* en ce qu'**elle comprend:
- de 50 mg à 500 mg de vitamine C, avantageusement 240 mg ou 120 mg ;
- de 10 mg à 500 mg de vitamine E, avantageusement 30 mg ;
- de 5 mg à 100 mg de zinc, avantageusement 12,5 mg ;
- de 0,2 mg à 10 mg de cuivre, avantageusement 1 mg ;
- de 2 mg à 50 mg de lutéine, avantageusement 10 mg ;
- de 0,5 mg à 10 mg de zéaxanthine, avantageusement 2 mg ;
- de 100 mg à 1000 mg de EPA, avantageusement 380 mg ou 172 mg ;
- de 100 mg à 1000 mg de DHA, avantageusement 190 mg ou 366 mg.

6. Composition selon l'une des revendications 1 à 5, pour son utilisation en tant que complément alimentaire en ophtalmologie.

7. Composition selon l'une des revendications 1 à 5, pour son utilisation dans le traitement des pathologies oculaires associées à une surexpression de la voie VEGF, avantageusement à une surproduction des récepteurs VEGFR.

8. Composition pour son utilisation selon la revendication 7, ***caractérisée* en ce que** la pathologie oculaire est choisie dans le groupe suivant : la DMLA exsudative, l'occlusion veineuse rétinienne, l'oedème maculaire, l'oedème maculaire diabétique, la myopie, la rétinopathie diabétique, le glaucome néovasculaire ou la néovascularisation cornéenne.

9. Composition pour son utilisation selon la revendication 7 ou 8, ***caractérisée* en ce qu'**elle est administrée à un sujet atteint des symptômes de la pathologie uniquement au niveau d'un oeil.

10. Composition selon l'une des revendications 1 à 5,
pour son utilisation dans :
- le maintien ou l'amélioration de la santé oculaire ; et/ou
- la prévention des maladies oculaires ; et/ou
- la modulation de l'expression et/ou de l'activité des récepteurs VEGFR des cellules oculaires.

11. Composition pour son utilisation selon la revendication 10, ***caractérisée* en ce qu'**elle est utilisée chez un sujet sain, avantageusement chez un sujet jeune à risque.

12. Composition pour son utilisation selon l'une des revendications 6 à 11, ***caractérisée* en ce que** la composition est administrée par voie orale.

13. Composition pour son utilisation selon l'une des revendications 6 à 12, ***caractérisée* en ce que** la composition est administrée de manière quotidienne.

14. Composition pour son utilisation selon l'une des revendications 6 à 13, ***caractérisée* en ce que** la composition se présente sous la forme d'une ou plusieurs capsules, avantageusement 2 capsules.

## Patentansprüche

1. Nutrazeutische ophthalmische Zusammensetzung, umfassend:
- Vitamine,
- Spurenelemente,
- Carotinoide,
- Omega-3-Fettsäuren und
- Resveratrol in einem Anteil von über 20 mg;
diese Zusammensetzung enthält keinen Safran.

2. Zusammensetzung gemäß Anspruch 1, ***dadurch gekennzeichnet, dass*** sie einen Anteil von mehr oder gleich 30 mg Resveratrol enthält, besser von über 30 mg, und noch besser von mehr oder gleich 50 mg.

3. Zusammensetzung gemäß einem der vorhergehenden Ansprüche ***dadurch gekennzeichnet, dass*** die
- Vitamine, alleine oder kombiniert, ausgewählt werden aus der Gruppe, die Vitamin C und Vitamin E enthält;
- die Spurenelemente, alleine oder kombiniert, ausgewählt werden aus der Gruppe, die Zink und Kupfer enthält;
- Karotinoide, alleine oder kombiniert, ausgewählt werden aus der Gruppe, die Zeaxanthin und Lutein enthält;
- Omega-3-Fettsäuren, alleine oder in Kombination aus Fischöl stammen und es von Vorteil ist, wenn sie aus der Gruppe ausgewählt werden, zu der Eicosapentaensäure (EPA) und Docosahexaensäure (DHA) gehören.

4. Zusammensetzung gemäß einem der vorhergehenden Ansprüche **dadurch gekennzeichnet, dass** sie enthält:
- Vitamin C;
- Vitamin E;
- Zink;
- Kupfer;
- Lutein;
- Zeaxanthin;
- Omega-3-Fettsäuren aus Fischöl, insbesondere EPA und DHA.

5. Zusammensetzung gemäß einem der vorhergehenden Ansprüche **dadurch gekennzeichnet, dass** sie enthält:
- 50 mg bis 500 mg Vitamin C, vorteilhafterweise 240 mg oder 120 mg;
- 10 mg bis 500 mg Vitamin E, vorteilhafterweise 30 mg;
- 5 mg bis 100 mg Zink, vorteilhafterweise 12,5 mg;
- 0,2 mg bis 10 mg Kupfer, vorteilhafterweise 1 mg;
- 2 mg bis 50 mg Lutein, vorteilhafterweise 10 mg;
- 0,5 mg bis 10 mg Zeaxanthin, vorteilhafterweise 2 mg;
- 100 mg bis 1000 mg EPA, vorteilhafterweise 380 mg oder 172 mg;
- 100 mg bis 1000 mg DHA, vorteilhafterweise 190 mg oder 366 mg.

6. Zusammensetzung gemäß einem der Ansprüche 1 bis 5 zur Verwendung als Nahrungsergänzung in der Ophtalmologie.

7. Zusammensetzung gemäß einem der Ansprüche 1 bis 5 zur Verwendung bei der Behandlung von Augenerkrankungen in Verbindung mit einer Überexpression des VEGF- Weges, vorteilhafterweise eine Überproduktion von VEGFR- Rezeptoren.

8. Zusammensetzung zur Verwendung gemäß Anspruch 7, ***dadurch gekennzeichnet, dass*** die Augenpathologie zur folgenden Gruppe gehört, exsudative AMD, Retinalvenenverschluss, Makulaödem, diabetisches Makulaödem, Myopie, diabetische Retinopathie, neovaskuläres Glaukom oder Hornhaut-Neovaskularisation.

9. Zusammensetzung zur Verwendung gemäß Anspruch 7 oder 8, ***dadurch gekennzeichnet, dass*** sie einem Patienten verabreicht wird, bei dem nur eine Auge von den pathologischen Symptomen betroffen ist.

10. Zusammensetzung gemäß einem der vorstehenden Ansprüche 1 bis 5, zur Verwendung bei der:
- Aufrechterhaltung oder Verbesserung der Augengesundheit; und/ oder
- Prävention von Augenerkrankungen; und/ oder
- Modulation der Expression und/ oder Aktivität der VEGFR- Rezeptoren der Augenzellen.

11. Zusammensetzung zur Verwendung gemäß Anspruch 10, ***dadurch gekennzeichnet, dass*** sie bei einem gesunden Patienten eingesetzt wird, am besten einem jungen Risikopatienten.

12. Zusammensetzung zur Verwendung gemäß einem der Ansprüche 6 bis 11 ***dadurch gekennzeichnet, dass*** die Zusammensetzung oral verabreicht wird.

13. Zusammensetzung zur Verwendung gemäß einem der Ansprüche 6 bis 12, ***dadurch gekennzeichnet, dass*** die Zusammensetzung täglich verabreicht wird.

14. Zusammensetzung zur Verwendung gemäß einem der Ansprüche 6 bis 13 ***dadurch gekennzeichnet, dass*** die Zusammensetzung die Form einer oder mehrere Kapseln hat, am besten 2 Kapseln.

## Claims

1. An ophthalmic nutraceutical composition comprising:
- vitamins;
- trace elements;
- carotenoids;
- omega-3 fatty acids; and
- resveratrol in a quantity of more than 20 mg,
said composition not comprising saffron.

2. A composition according to claim 1, ***wherein*** it comprises a quantity of resveratrol that is equal to or greater than 30 mg, advantageously greater than 30 mg, preferably equal to or greater than 50 mg.

3. A composition according to any of the preceding claims, ***wherein:***
- the vitamins, alone or in combination, are selected from the group comprising vitamin C and vitamin E;
- the trace elements, alone or in combination, are selected from the group comprising zinc and copper;
- the carotenoids, alone or in combination, are chosen from the group comprising zeaxanthin and lutein;
- the omega-3 fatty acids, alone or in combination, come from fish oil and are advantageously selected from the group comprising eicosapentaenoic acid (EPA) and docosahexaenoic acid (DHA).

4. A composition according to any of the preceding claims, ***wherein*** it comprises:
- vitamin C;
- vitamin E;
- zinc;
- copper;
- lutein;
- zeaxanthin; and
- omega-3 fatty acids from fish oil, particularly EPA and DHA.

5. A composition according to any of the preceding claims, ***wherein*** the composition comprises:
- 50 mg to 500 mg of vitamin C, advantageously 240 mg or 120 mg;
- 10 mg to 500 mg of vitamin E, advantageously 30 mg;
- 5 mg to 100 mg of zinc, advantageously 12.5 mg;
- 0.2 mg to 10 mg of copper, advantageously 1 mg;
- 2 mg to 50 mg of the lutein, advantageously 10 mg;
- 0.5 mg to 10 mg of zeaxanthin, advantageously 2 mg;
- 100 mg to 1000 mg of EPA, advantageously 380 mg or 172 mg; and
- 100 mg to 1000 mg of DHA, advantageously 190 mg or 366 mg.

6. A composition according to any of claims 1 to 5, for use as a food supplement in ophthalmology.

7. A composition according to any of claims 1 to 5, for use to treat an eye pathology associated with an overexpression of the VEGF pathway, advantageously with an overproduction of VEGFR receptors.

8. A composition for its use according to claim 7, ***wherein*** the eye pathology is selected from exudative AMD, retinal venous occlusion, macular edema, diabetic macular edema, myopia, diabetic retinopathy, neovascular glaucoma or corneal neovascularization.

9. A composition for its use according to claim 7 or 8, ***wherein*** it is administered to a subject suffering from symptoms of said pathology only in one eye.

10. A composition according to any of claims 1 to 5, for use in:
- maintaining or improving eye health; and/or
- prevention of eye diseases; and/or
- modulation of the expression and/or of the activity of the VEGFR receptors of the eye cells.

11. A composition for its use according to claim 10, wherein it is used in a healthy subject, advantageously a young subject at risk.

12. A composition for its use according to any of claims 6 to 11, ***wherein*** the composition is administered orally.

13. A composition for its use according to any of claims 6 to 12, ***wherein*** the composition is administered daily.

14. A composition for its use according to any of claims 6 to 13, ***wherein*** the composition is in the form of one or more capsules, advantageously 2 capsules.
